(19) Europäisches Patentamt
European Patent Office
Office européen des brevets

(11) Publication number: 0 251 229 B1

(12) EUROPEAN PATENT SPECIFICATION

(45) Date of publication of the patent specification: 04.04.90

(51) Int. Cl.⁴: C07F 9/10

(21) Application number: 87109189.8

(22) Date of filing: 26.06.87

(54) **Polymerizable beta-glycerophospholipid and method for production thereof.**

(30) Priority: 27.06.86 JP 150941/86

(43) Date of publication of application: 07.01.88 Bulletin 88/1

(45) Publication of the grant of the patent: 04.04.90 Bulletin 90/14

(84) Designated Contracting States: BE CH DE FR GB IT LI NL SE

(56) References cited:
EP-A- 0 032 622
EP-A- 0 036 155
EP-A- 0 107 120
DE-A- 2 647 395

PATENT ABSTRACTS OF JAPAN, vol. 9, no. 200 (C-298)[1923], 16th August 1985; & JP - A - 60 67489 (TOYO SODA KOGYO K.K.) 17-04-1985
CHEMICAL ABSTRACTS FORMULA INDEX, C16-Z, vol. 89, July-December 1978, page 2278F, Column 1, line 100; column 2, line 28, Columbus, Ohio, US;
J. LIEBIG "Annalen der Chemie", vol. 709, 1967, pages 226-230, formula, line 10, page 230, Publication Chemistry GmbH, Weinheim
Ann. N.Y. Acad. Sci., 446, pages 282-293, 1985

(73) Proprietor: Terumo Kabushiki Kaisha, No. 44-1, Hatagaya 2-chome Shibuya-ku, Tokyo 151 (JP)

(72) Inventor: Yoshioka, Hiroshi, 2517, Ohmiya, Fujinomiya-shi Shizuoka-ken(JP)
Inventor: Suzuki, Kazuhiko, 421-3, Kuroda, Fuji-shi Shizuoka-ken(JP)

(74) Representative: Henkel, Feiler, Hänzel & Partner, Möhlstrasse 37, D-8000 München 80(DE)

EP 0 251 229 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to a novel polymerizable glycerophospholipid and a method for the production thereof. More particularly, it relates to a polymerizable beta-glycerophospholipid capable of forming a macromolecular liposome of highly desirable stability and a method for the production thereof.

Description of the Prior Art:

At present various attempts are being made to materialize supply of medicinal substances, enzymes, etc. sealed in microcapsules as finished medicines and drugs. Microcapsules which have hemoglobin sealed therein, for example, are handled as artificial red blood cells.

The classical methods employed for the microcapsulation of this nature have been directed to the capsulation of macromolecular compounds by the technique of emulsification or to the capsulation effected by the technique of interfacial polycondensation and inevitably attended by the formation of a polymer (polyamide). These methods, however, have entailed problems fatally detrimental to safe use of the products of capsulation as medicines and drugs, such as the conspicuous toxicity inherent in a polymer used as the material for capsulation, the toxicity inherent in an organic solvent indispensably used during the synthesis of the polymer and consequently suffered to remain in produced capsules, and the unduly large particle diameter of produced capsules (several µm to 1,000 µm) such as to induce disorders like thrombosis.

Incidentally, the microcapsulation of medicinal substances, enzymes, hemoglobin, etc. is aimed mainly at enabling the medicinal substances, enzymes, hemoglobin, etc. which are inherently unstable in living systems to retain their ability for a long time and the effects thereof to last for a long time. The material to be used in microcapsulating the medicines and drugs intended for use in living systems is expected to fulfil the requirement that it should manifest only insignificant toxicity on living systems, permit minimization of the capsules to be produced, and enables the capsules to retain ample stability within living systems.

The liposome i.e. the aggregate of microfine spheres formed in water by various phospholipids which are main components of biomembranes, has been known to fulfil this requirement to a fairly large extent. Thus, the use of liposome as the material for microcapsules has been drawing growing attention.

The liposome which use natural phospholipids in the unaltered form, however, has a short life and produces an unstable interaction particularly with living cells. In the field of the drug delivery using the liposome as model for the identification of cells, as a model for the classification of interactions between cells, and as a carrier for the deposition of medicines and drugs, therefore, numerous studies are being made in search of a stable liposome. The most efficient means so far developed by these studies consists in high polymerization of the liposome.

The high polymerization of the liposome is aimed at stabilizing the structure of dyadic membranes and consequently that of endoplasmic reticula by the formation of lipid molecules. Specifically this high polymerization is mainly effected by the method which comprises incorporating in lipid molecules a functional group possessing a polymerizing ability thereby preparing a liposome as the monomer subsequently causing polymerization of lipids in the liposome membrane.

Various species of polymeric liposome-forming lipids of the class described above have been synthesized. From the viewpoint of the drug delivery, those polymeric liposome-forming lipids possessing the structure of phospholipids, particularly glycerophospholipids represented by phosphatidyl choline, which are main components of the lipids in the biomembranes are most promising. The species of the polymerizable phosphatidyl choline heretofore known to the art possess a conjugate diyne or a conjugate diene in two acyl chains as a polymerizable functional group. The acyl chain or fatty acid possessing the conjugate diyne or conjugate diene is obtainable solely by a pure organic chemical means of synthesis based on an extremely meticulous molecular design. Thus, it cannot be easily synthesized in quantities fitting commercial production. Moreover, the polymerizable phosphatidyl choline using this fatty acid is extremely expensive. The production of this polymerizable phosphatidyl choline is effected, for example, by a method which is published in Journal of American Chemical Society, 106 1627 (1984) comprises first synthesizing an unsaturated fatty acid possessing a polymerizable functional group and esterifying this acid with the hydrolyzate of natural phosphatidyl choline. The polymerizable phosphatidyl choline thus obtained, therefore, invariably acquires an alpha-phosphatidyl choline structure which, similarly to the structure of natural phosphatidyl choline, has acyl chains attached one each to the alpha-position and the beta-position and a phosphoryl choline group to the alpha'-position, respectively of a glycerol skeleton represented by the general formula:

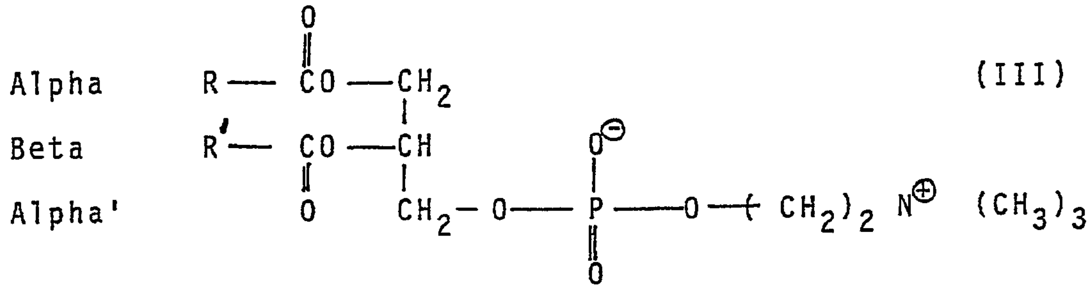

(III)

wherein R and R′ independently stand for a saturated or unsaturated hydrocarbon group.

It has been demonstrated, however, as recently reported in O'Brien et al., Ann. N.Y. Acad. Sci., 446 282 (1985) that the alpha-phosphatidyl choline of the foregoing description in the state of liposome has the two acyl chains deviated by an interval of about four atoms in the direction perpendicular to the plane of liposome membrane (Figs. 2 and 3) and the polymerizable functional groups in the two acyl chains are consequently displaced so that no polymerization reaction is allowed to occur between the alpha-chain and the beta-chain and, as the result, the quantum yield of the photopolymerization reaction is lower by several thousand times than that obtainable by the symmetrical quanternary ammonium salt type lipid which, in the state of liposomes, has the polymerizable functional groups in the two acyl chains coincide with each other (Fig. 4). The polymerizable alpha-phosphatidyl choline of the nature described above, because of the structural asymmetry thereof, necessitates a huge amount of energy for the polymerization reaction thereof in the state of liposome and the macromolecular liposome consequently obtained is deficient in stability.

In Chemical Abstracts, 89, page 2278, DE-A 2 647 395 and J. Liebig "Annalen der Chemie", vol. 709, 1967, pp. 226–230 ethylenically unsaturated beta-glycerophospholipids are described. These compounds do not show functional conjugate triene groups and may not be used to produce macromolecular liposomes of improved stability. Further, compounds of a less related structure are known from Patent Abstracts of Japan, vol. 9, no. 200, August 1985, and EP-A 0 107 120.

An object of this invention, therefore, is to provide a novel polymerizable glycerophospholipid and a method for the production thereof.

Another object of this invention is to provide a polymerizable glycerophospholipid capable of forming a macromolecular liposome of outstanding stability and a method for the production thereof.

Still another object of this invention is to provide a polymerizable glycerophospholipid such that the monomer liposome formed thereof can be easily polymerized under mild conditions and a method for the production of the polymerizable glycerophospholipid.

A further object of this invention is to provide a polymerizable glycerophospholipid capable of forming a macromolecular liposome of high bioadaptability and a method for the production of the polymerizable glycerophospholipid.

The objects described above are accomplished by a polymerizable beta-glycerophospholipid represented by the following structural formula I:

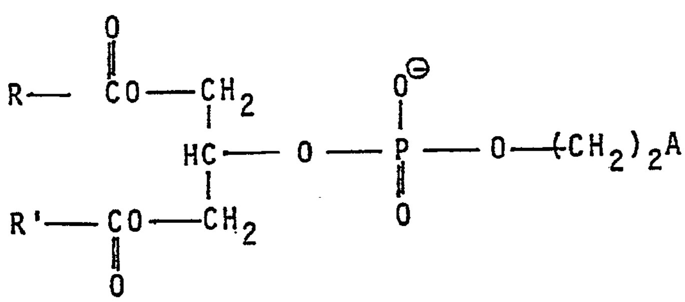

(I)

wherein R and R′ independently stand for an aliphatic hydrocabon group possessing a conjugate triene as a polymerizable functional group and A stands for $N^{\oplus}R^1_3$ wherein $R^1$ stands for methyl group or ethyl group, or 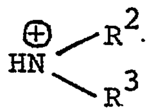 wherein $R^2$ and $R^3$ independently stand for hydrogen atom, methyl group, or ethyl group.

The present invention also concerns a polymerizable beta-glycerophospholipid of the structural formula wherein R and R′ independently stand for an aliphatic hydrocarbon group containing a conjugate triene and having 12 to 26 carbon atoms, preferably $CH_3(CH_2)_3CH{=}CHCH{=}CHCH{=}CH(CH_2)_7-$. This invention further concerns a polymerizable beta-glycerophospholipid of the structural formula, wherein A stands for $N^{\oplus}R^1_3$. This invention also concerns a polymerizable beta-glycerophospholipid of the structural formula, wherein $R^1$ stands for methyl group.

The objects desribed above are further accomplished by a method for the production of a polymerizable beta-glycerophospholipid represented by the structural formula I:

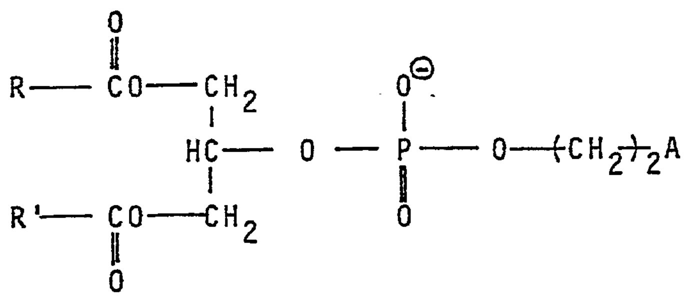

(I)

wherein R and R′ independently stand for an aliphatic hydrocarbon group possessing a conjugate triene as a polymerizable functional group and A stands for $N^{\oplus}R^1_3$ wherein $R^1$ stands for a methyl group or an ethyl group 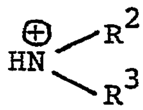 wherein $R^2$ and $R^3$ independently stand for a hydrogen atom, a methyl group, or an ethyl group, which method comprises causing a 1,3-diglyceride represented by the following formula II:

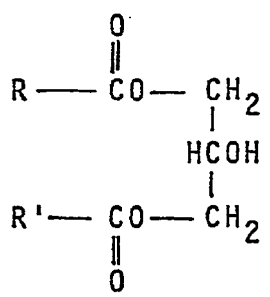

(II)

wherein R and R′ independently stand for an aliphatic hydrocarbon group possessing a conjugate triene as a polymerizable functional group, to react with a 2-halo-2-oxo-1,3,2-dioxaphosphoran in the presence of a tertiary amine and subsequently causing the product of this reaction to react with an amine or ammonia.

This invention also concerns a method for the production of a polymerizable beta-glycerophospholipid of the formula I wherein R and R′ independently stand for an aliphatic hydrocarbon group containing a conjugate triene and having 12 to 26 carbon atoms.

This invention also discloses a method for the production of a polymerizable beta-glycerophospholipid, wherein the 1,3-diglyceride is a species represented by the following structural formula IIa:

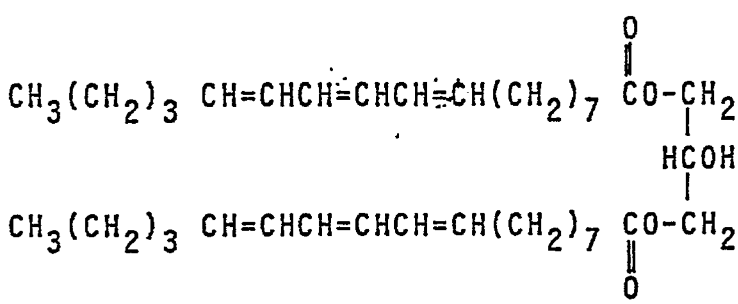

(IIa)

The present invention further discloses a method for the production of a polymerizable beta-glycerophospholipid, wherein the amine is trimethyl amine or triethyl amine, preferably trimethyl amine. This invention also discloses a method for the production of a polymerizable beta-glycerophospholipid, wherein the 2-halo-2-oxo-1,3,2-dioxaphosphoran is 2-chloro-2-oxo-1,3,2-dioxaphosphoran. This invention further discloses a method for the production of a polymerizable beta-glycerophospholipid, wherein the 1,3-diglyceride represented by the formula II is obtained by the reaction of an unsaturated fatty anhydride with glycidol in the presence of a quaternary ammonium salt. The present invention also discloses a method for the production of a polymerizable beta-glycerophospholipid, wherein the unsaturated fatty anhydride is eleostearic anhydride. Further, the present invention discloses a method for the production of a polymerizable beta-glycerophospholipid, wherein the eleostearic anhydride is produced from eleostearic acid contained in tung oil fatty acid by the use of a haloformic ester.

BRIEF DESCRIPTION OF THE DRAWINGS

Fig. 1 is a diagram illustrating the molecular structure of the polymerizable beta-phosphatidyl choline on the present invention in the state of liposome formation,

Figs. 2–4 are diagrams illustrating the molecular structures of the conventional liposome-forming polymerizable lipids in the state of liposome formation,

Fig. 5 is a chart of the $^1$H-NMR spectrum of 1,3-dieleostearoyl glyceride,

Fig. 6 is a chart of the infrared absorption spectrum of a typical polymerizable beta-glycerophospholipid of the present invention (compound of formula Ia),

Fig. 7 is a chart of the $^1$H-NMR spectrum of a typical polymerizable beta-glycerophospholipid of the present invention (compound of formula Ia),

Fig. 8 is a chart of the ultraviolet light absorption spectrum showing the degree of the polymerization effected by irradiation of ultraviolet light on the liposome to be produced from the polymerizable beta-glycerophospholipid of the present invention, and

Fig. 9 is a graph showing the relation between the ethanol concentration enough for destruction of macromolecular liposome and the turbidity of a liposome suspension.

Now, the present invention will be described in detail below with reference to preferred embodiments.

One specific compound preferably representing the polymerizable beta-glycerophospholipid of the present invention is 1,3-dieleostearoyl glycero-2-phospho choline represented by the following structural formula Ia:

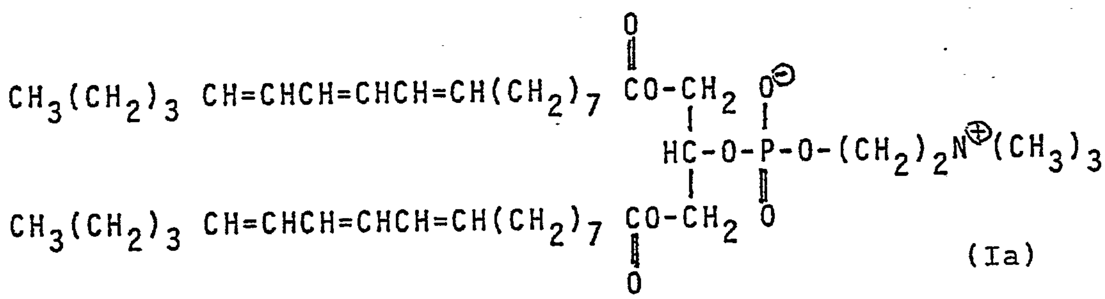

(Ia)

As is clearly noted from this structural formula (Ia), the polymerizable phosphatidyl choline which is one specific example of the polymerizable beta-glycerophospholipid of the present invention, similarly to the natural phosphatidyl choline, is an amphiphilic compound possessing a hydrophobic nonpolar moiety containing two long aliphatic acyl chains and a hydrophilic polar moiety containing a phosphoryl choline group. When this compound is dispersed in an aqueous medium, it readily forms the so-called liposome, i.e. a closed endoplasmic reticulum containing a dyadic membrane of lipid having the hydrophilic polar moiety thereof disposed externally (an inner aqueous layer and an outer aqueous medium) and the hydrophobic nonpolar moiety thereof disposed internally around an aqueous layer. Any of the polymerizable beta-glycerophospholipids represented by the formula I similarly forms a corresponding liposome.

The polymerizable beta-phosphatidyl choline represented by the structural formula (Ia) is one species of the polymerizable beta-glycerophospholipid of this invention excelling in symmetry and represented by the general formula I:

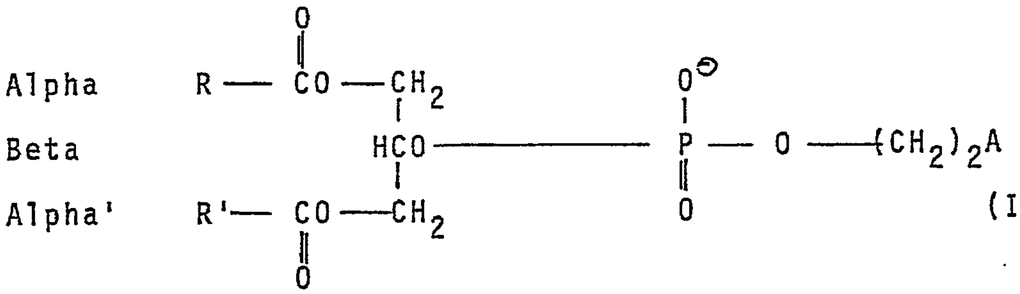

(I)

wherein R and R′ independently stand for an aliphatic hydrocarbon group possessing a conjugate triene as a polymerizable functional group and A stands for $N^{\oplus}R^1_3$ wherein $R^1$ stands for methyl group or ethyl group or 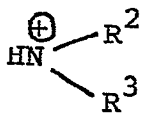 wherein $R^2$ and $R^3$ independently stand for hydrogen atom, methyl group, or ethyl group and has aliphatic acyl groups, specifically eleostearoyl gorups originating in eleostearic acid, attached one each to the alpha-position and the alpha′-position and a phosphoryl choline group attached to the beta-position, respectively of the glycerol skeleton. Unlike the natural phosphatidyl choline possessing the structure of an alpha-phosphatidyl choline represented by the following formula III:

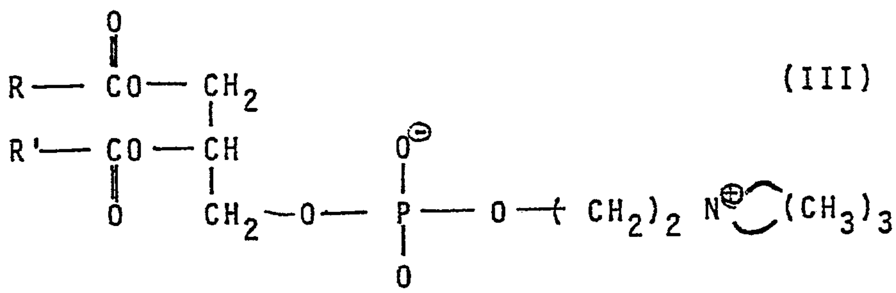

(III)

wherein R and R′ independently stand for a saturated or unsaturated aliphatic hydrocarbon group, or the synthetic phosphatidyl choline derived from the natural phosphatidyl choline, therefore, the polymerizable alpha-phosphatidyl choline of the structural formula (I) has the two acyl chains thereof disposed as not deviated in the direction perpendicular to the plane of the liposome membrane (Fig. 1.). As the result, the reaction between the eleostearoyl group (alpha-chain) present at the alpha-position of the glycerol skeleton and the polymerizable functional group (conjugate triene) of the eleostearoyl group (alpha′-chain) present at the alpha′-position of the glycerol skeleton comprises the polymerization reaction between the alpha-chain and the alpha′-chain in addition to the alpha-chain-alpha-chain reaction and the alpha′-chain-alpha′-chain reaction, with the result that the energy required for the polymerizatoin reaction is decreased and the stability of the produced macromolecular liposome is increased.

The polymerizable beta-phosphatidyl choline of the structural formula Ia according to the present invention possesses an acyl group originating in eleostearic acid and represented by the general formula IV:

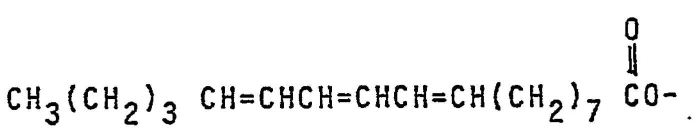

(IV)

The eleostearic acid is a natural unsaturated fatty acid possessing conjugate double bonds (conjugate triene) one each at the 9, 11, and 13 positions as represented by the general formula IVa:

$$CH_3(CH_2)_3\ CH{=}CHCH{=}CHCH{=}CH(CH_2)_7\ COOH \qquad \text{(IVa)}$$

It occurs as a glyceride in tung oil and accounts for 80 to 95% by weight of the mixed fatty acids of the tung oil. In the present invention, therefore, as described later, the conjugate triene groups of the eleostearic acid are utilized in their unaltered form as polymerizable functional groups. This invention does not require the polymerizable functional groups to be synthesized by a process fo organic chemistry.

Moreover, since the conjugate trienes have a maximum absorption wavelength ($\lambda max$) of 272 nm and fall in a larger wavelength region than the conjugate diene ($\lambda max$=257 nm) and on a lower energy side, the polymerization reaction can be initiated as with the ultraviolet light of lower energy. Further, as compared with the polyacetylene type lipid of a rigid structure possessing conjugate triple bonds within the molecule thereof which is enabled to polymerize only in the crystalline state short of the state of gel-crystalline phase transition [D. Chapman et al., Biochim. Biophys, Acta, 602, 57 (1980); ibid 602, 213 (1980)], the polymerizable beta-phosphatidyl choline of the structural formula Ia possesses the conjugate trienes as polymerizable functional groups and has the acyl chains of a relatively soft structure and, therefore, is enabled by the electromagnetic wave to initiate the polymerization reaction in any state either above or below the phase transition temperature.

Of course, the polymerizable beta-glycerophospholipid of the present invention is not limited to the polymerizable beta-phosphatidyl choline represented by the structural formula Ia. All other compounds represented by the general formula I are embraced by this invention, no matter whether they possess skeletons such as of phosphatidyl ethanolamine instead of the skeleton of phosphatidyl choline.

The polymerizable beta-glycerophospholipid represented by the general formula I and possessed of the properties described above can be prepared by the method of the present invention to be described below. It should be noted, however, that the method of this invention can be applied not merely to the preparation of the polymerizable beta-phosphatidyl choline represented by the structural formula Ia but also to the preparation of various polymerizable beta-glycerophospholipids represented by the formula I such as, for example, phosphatidyl choline possessing conjugate trienes as polymerizable functional groups in the acyl chains and having $N^{\oplus}(CH_3)_3$ as the substituent A in the general formula I and phosphatidyl ethanolamine having $N^{\oplus}H_3$ as the substituent A mentioned above.

To be specific, the polymerizable beta-glycerophospholipid represented by the general formula I is prepared by causing a 1,3-diglyceride represented by the following general formula II:

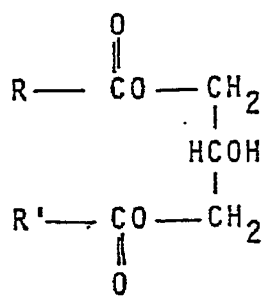

(II)

wherein R and R′ independently stand for an aliphatic hydrocarbon group possessing a conjugate triene as a polymerizable functional group, to undergo a condensation reaction with a 2-halo-2-oxo-1,3,2-dioxaphosphoran represented by the following formula V:

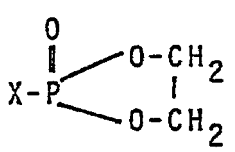

(V)

wherein X stands for a halogen atom, in the presence of a tertiary amine thereby producing a compound represented by the following formula VI:

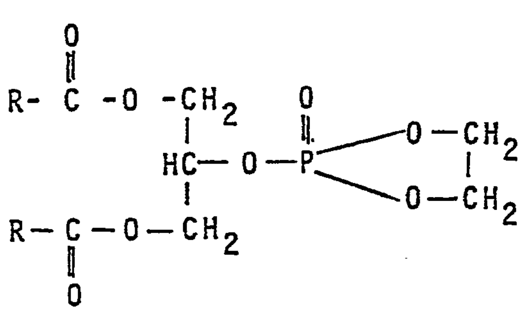

(VI)

and subsequently causing this produced compound to react with and amine or ammonia. The last reaction

gives rise to phosphatidyl choline [A = $N^{\oplus}(CH_3)_3$] when trimethylamine is used therein or phosphatidyl ethanol amine (A = $N^{\oplus}H_3$) when ammonia is used therein. When some other amine than trimethyl amine is used in this reaction, there is obtained a corresponding compound.

Now, the method of this invention will be described more specifically below with reference to the preparation of 1,3-dieleostearoyl glycero-2-phosphoryl choline represented by the structural formula Ia.

Typical examples of the 2-halo-2-oxo-1,3,2-dioxaphosphoran represented by the formula V include 2-chloro-2-oxo-1,3,2-dioxaphosphoran, 2-bromo-2-oxo-1,3,2-dioxaphosphoran, and 2-fluoro-2-oxo-1,3,2-dioxaphosphoran. Among other specific compounds enumerated above, 2-chloro-2-oxo-1,3,2-dioxaphosphoran proves to be particularly desirable. This particular compound is synthesized by following the procedure described proposed by R.S. Edmundson et al. in Chem Ind. (London), 1829 (1962), with necessary modifications and then refined by vacuum distillation before it is put to use.

The amount of the compound of the general formula V and that of the tertiary amine such as trimethyl amine or triethyl amine to be used as an agent for the removal of hydrochloric acid may be equal in mol to that of the 1,3-diglyceride of the general formula II. The reaction is carried out in a thoroughly dehydrated solvent such as chloroform or tetrahydrofuran under the atmosphere of an inert gas at a temperature in the range of 0°C to 50°C, preferably 40°C to 50°C. Generally, the reaction time is in the range of 1 to 10 hours. Thus, the compound of the formula IV is obtained quantitatively. When 1,3-dieleostearoyl glyceride of the following structural formula IIa:

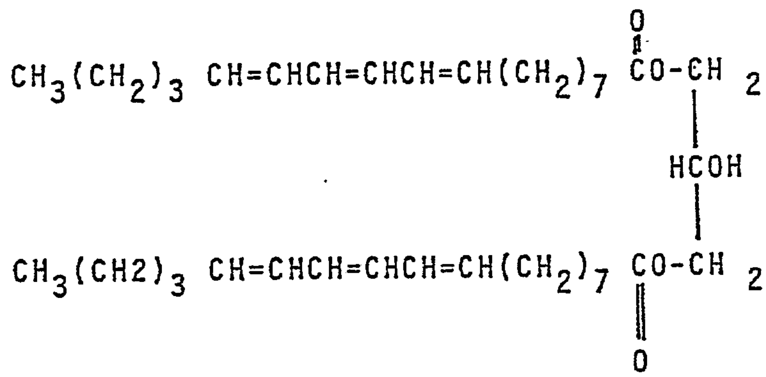
(IIa)

is used as a compound of the formula II, for example, a compound of the following structural formula VIa:

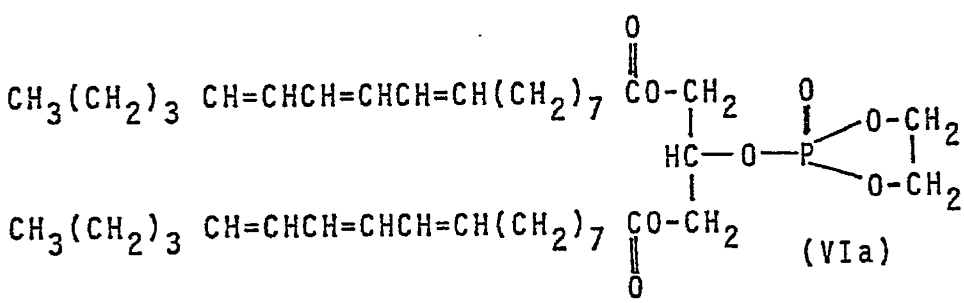
(VIa)

is obtained as an intermediate.

Then, the reaction of the compound of the formula VI with an amine such as, for example, trimethyl amine, or with ammonia is carried out in a dehydrated polar solvent such as dimethyl formamide or acetonitrile at a temperature in the range of room temperature to 60°C, preferably 50°C to 60°C, for a period in the range of several ours to some tens of hours, preferably 10 to 20 hours. The amount of the amine such as trimethyl amine or ammonia to be used for the reaction has only to be at least equal in mol to that of the compound of the formula VI. Preferably, this amount is not less than 5 times that of the aforementioned compound. In the preparation of the polymerizable beta-phosphatidyl choline of the formula I such as, for example, the compound of the structural formula Ia, to be obtained after completion of the reaction with trimethyl amine, 1,3-dieleostearoyl glycero-2-phosphoryl choline of the structural formula Ia can be purified by the silica gel column chromatography using a chloroform/methanol mixed solvent.

The 1,3-diglyceride of the formula II which is used in the method of production described above has heretofore been produced by the method proposed by A.P.J. Mank et al. in Chem. Phys. Lipids, 16, 107 (1976). This method required epichlorohydrin to undergo two steps of reaction, first with a sodium salt of fatty acid and then with a fatty acid and further entails an operation for the conversion of 1,2-diglyceride into 1,3-diglyceride and, therefore, proves to be disadvantageous where there is used an unsaturated fatty acid of a low melting point is used. In the present invention, the 1,3-diglyceride of the formula II can be obtained in one step by the reaction of an unsaturated fatty acid anhydride with glycidol. This reaction is accelerated in the presence of a quaternary ammonium salt.

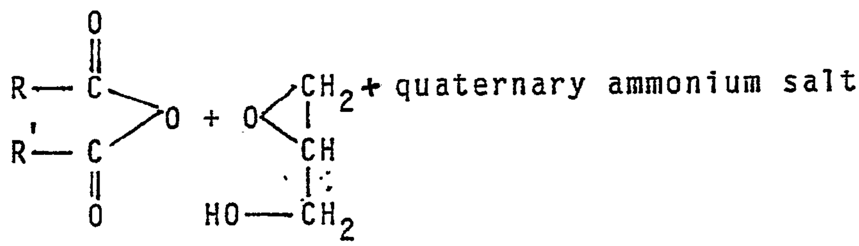


(II)

In this case, the amount of the unsaturated fatty acid anhydride to be used is equal in mol to that of the glycidol. This reaction is carried out in a dehydrated solvent such as tetrahydrofuran or chloroform in the atmosphere of an inert gas such as nitrogen or argon by the addition of a quaternary ammonium salt such as tetraethyl ammonium bromide or tetra-n-butyl ammonium bromide at a temperature in the range of 40° to 60°C for a period in the range of one to five days. The amount of the quaternary ammonium salt to be added as the catalyst may be in the range of $1/10$ to $1/100$, preferably about $1/25$, in molar ratio relative to the amount of the glycidol. When the 1,3-dieleostearoyl glyceride of the structural formula IIa is to be obtained, it suffices to resort to the reaction of the eleostearic anhydride represented by the structural formula VII:

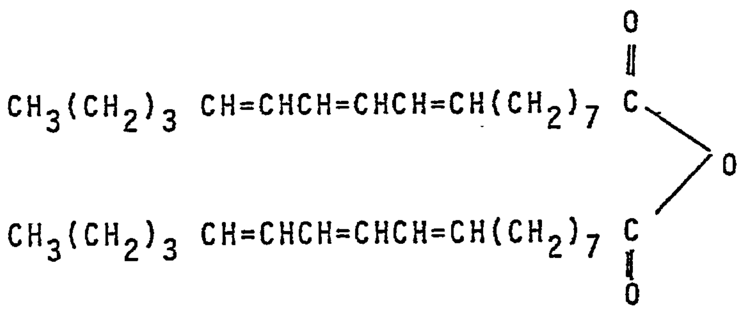

(VII)

with glycidol under the following conditions. The 1,3-dieleostearoyl glyceride (IIa) which is the reaction product can be easily formed by repeating recrystallization of the reaction mixture from hexane.

The eleostearic anhydride (VII) is derived from the eleostearic acid (IVa) which is a natural unsaturated fatty acid. As described above, the eleostearic acid is present as a glyceride in tung oil and accounts for 80 to 95% by weight of the mixed fatty acids of the tung oil. The tung oil fatty acid obtained by the hydrolysis of tung oil contains not less than 60% by weight, preferably not less than 80% by weight, of eleostearic acid and the balance of saturated fatty acids, oleic acids, linoleic acid, etc. Thus tung oil fatty acid may be used in its unaltered form as a natural unsaturated fatty acid or, when desired, may be refined as by recrystallization and/or column chromatography for isolation of eleostearic acid (IIa). The production of this eleostearic anhydride (VII) is effected by gradually adding to a dehydrated solution of equimolar amounts of the eleostearic acid (IIa) and a tertiary amine such as trimethyl amine or triethyl amine in tetrahydrofuran or chloroform, a haloformic ester such as ethyl bromoformate, propyl chloroformate, ethyl bromoformate, or propyl bromoformate in an amount equal in mol to the amount of the eleostearic acid at a temperature in the range of –20° to 0°C, allowing the ensuing reaction to proceed at the same temperature for one to two hours, continuing the reaction further at room temperature for 20 to 30 minutes, gradually adding to the resultant reaction mixture a tertiary amine salt of eleostearic acid in an amount equal in mol to the amount of the first eleostearic acid at a temperature in the range of –20° to 0°C, and further continuing the esuing reaction at room temperature for 8 to 20 hours. The eleostearic anhydride (VII) consequently obtained can be sufficiently purified by filtration and washing with water. By this method, the eleostearic anhydride is obtained substantially stoichiometrically without being affected by the conjugate triene group of the eleostearic acid (IIa).

By the conventional method adopted for the production of a liposome from a natural phosphatidyl choline as proposed by D. Papahakjopoulos et al., in Biochim. Biophys. Acta., 135, 639 (1967), the polymerizable beta-glycerophospholipid obtained by the method for production according to this invention is enabled to form a liposome in water. This monomer liposome can be used as a carrier for medicinal substances, enzymes, hemoglobin, etc.

From the monomer liposome obtained as described above, a macromolecular liposome can be formed by exposing this monomer liposome to an ultraviolet light, a gamma ray, or an electron beam thereby inducing polymerization of the particularly when the polymerizable beta-glycerophospholipid happens to be 1,3-dieleostearoyl glycero-2-phosphoryl choline of the structural formula Ia according to the present invention, the formation of the macromolecular liposome is obtained even by the ultraviolet light of low energy because the three double bonds in the two eleostearoyl groups are readily polymerized by this radiation. Owing to the high polymerization effected in the manner described above, the stability of liposome is enhanced. This macromolecular liposome can also be used as a carrier for medicinal substances, enzymes, hemoglobin, etc. When a given substance to be carried is of hydrophilic quality, it is carried as sealed in the inner aqueous phase of the monomeric or macromolecular liposome. Conversely when a given substance is of hydrophobic quality, it is carried on the aliphatic moiety of the monomeric or macromolecular liposome.

Now, the present invention will be described more specifically below with reference to working examples.

Example

Synthesis of eleostearic anhydride

A solution of 22.6 g of eleostearic acid in 400 ml of distilled tetrahydrofuran (THF) and 15.4 ml of triethyl amine added thereto were cooled to –20°C. To the resultant cooled solution, a solution of 9.2 ml of ethyl chloroformate in 300 ml of THF was added dropwise at –20°C over a period of 1.5 hours, with the ensuing reaction left continuing at –20°C for two hours. The temperature of the reaction solution was elevated to room temperature (20°C) and the reaction was further continued for 20 minutes at this temperature.

Then, the temperature was lowered to –20°C. To the resultant cooled reaction solution, a solution of 22.6 g of eleostearic acid and 15.4 ml of triethyl amine in 400 ml of THF was added dropwise at –20°C over period of 1.5 hours. The resultant mixture was left undergoing a reaction at room temperature for 15 hours. After completion of the reaction, the white insoluble precipitate formed in the reaction system was removed by filtration and the filtrate was distilled under a vacuum for expulsion of the solvent. The residue of the distillation was dissolved in 1.5 liters of diethyl ether, washed with water, separated, and dried over a anhydrous sodium sulfate. The resultant solution was filtered for removal of the desiccant, then distilled under a vacuum to expel the solvent, and subsequently dried under a vacuum. Consequently, eleostearic anhydride was obtained in a yield of 98%.

Synthesis of 1,3-dieleostearoyl glyceride (IIa)

A solution of 25.0 g of eleostearic anhydride and 3.44 g of glycidol in 75 ml of distilled THF and 0.60 g of tetra-n-butyl ammonium bromide added thereto were left undergoing a reaction under an atmosphere of nitrogen at 50°C for five days. The resultant reaction solution was distilled under a vacuum for removal of the solvent. The residue of distillation was dissolved in 400 ml of hexane, washed with water, separated, then recrystallized cold five times from hexane, and subsequently dried under a vacuum, to obtain 1,3-dieleostearoyl glyceride (IIa) in a yield of 32%. The identification of the product was conducted by the $^1$H-NMR spectrum (fig. 5).

Synthesis of 2-chloro-2-oxo-1,3,2-dioxaphoshoran (V)

To a solution of 200 g of reagent-grade phosphorus trichloride in 160 ml of distilled dichloromethane, 80 ml of distilled ethylene glycol was added dropwise at room temperature in the atmosphere of nitrogen gas over a period of 1.5 hours. The resultant solution was left undergoing a reaction at room temperature for 15 hours. Then, it was distilled under a vacuum for removal of the solvent and then purified by vacuum distillation (boiling point 63°C to 65°C/40 mmHg) to obtain 2-chloro-1,3,2-dioxaphosphoran as an intermediate. This intermediate was dissolved in 120 ml of distilled benzene and aerated with oxygen at room temperature for 15 hours. The resultant aerated solution was distilled under a vacuum for removal of the solvent and then purified by distillation under a vacuum, to obtain 2-chloro-2-oxo-1,3,2-dioxaphosphoran (V) having a boiling point of 103°C to 104°C/3 mmHg in a yield of 52%.

Production of polymerizable beta-phosphatidyl choline (Ia)

To a solution of 8.3 g of 1,3-dieleostearoyl glyceride and 1.4 g of triethyl amine in 200 ml of distilled chloroform, a solution of 2.3 g of 2-chloro-2-oxo-1,3,2-dioxaphosphoran was added dropwise at 10°C under the atmosphere of nitrogen gas over a period of 30 minutes. After completion of the dropwise addition, the resultant reaction solution was left undergoing a reaction at 40°C for 15 hours. The resultant reaction solution was distilled under a vacuum for removal of the solvent. The distillate was dissolved in 50 ml of distilled dimethyl formamide. The solution and 5 ml of trimethyl amine were placed in a pressure reaction tube and left undergoing a reaction at 50°C for 20 hours. The resultant reaction solution was distilled under a vacuum for removal of the solvent. The distillate was dissolved in diethyl ether and the resultant solution was filtered for removal of insolubles. Then, the filtrate was distilled under a vacuum for removal of the solvent. The residue was purified by silica gel column chromatography using a chloroform-methanol mixed solvent. Consequently, a polymerizable beta-phosphatidyl choline of the formula of Ia was obtained in a yield of 61%. The identification of product was conducted by IR spectrum (Fig. 6) and $^1$H-NMR spectrum (Fig. 7).

Referential experiment

Example of production of liposome from polymerizable beta-phosphatidyl choline

A solution of 50 ml of the polymerizable beta-phosphatidyl choline of the formula Ia in 5 ml of $CHCl_3$ was placed in an eggplant-shaped flask and treated with a rotary evaporator for thorough removal of the solvent, with the result that a film of lipid was formed on the bottom surface of the eggplant-shaped flask. The film of lipid and 10 ml of nitrogen-aerated distilled water added thereto were sonically treated with an ultrasonic cleaning device under the atmosphere of nitrogen gas as kept cooled with ice for 5 minutes. The solution resulting from the treatment was negative stained with phosphotungstic acid and was observed under a transmission type electron microscope at 100,000 magnifications. Consequently, formation of a liposome film about 50 Å in thickness was confirmed.

Example of polymerization of liposome

A suspension of the monomer liposome obtained in the preceding example of production of liposome was placed in a quartz test tube and irradiated with the ultraviolet light issuing from a 32-W low-pressure mercury vapor lamp disposed at a distance of 10 cm at room temperature in the atmosphere of nitrogen gas. With the elapse of the time of irradiation, the absorbance of the ultraviolet absorption ($\lambda max$ = 272 nm) due to the conjugate trienes was observed to decrease, clearly indicating a progress of polymerization.

The macromolecular liposome obtained consequently was placed in an aqueous ethanol solution to determine the relation between the ethanol concentration and the turbidity of the liposome suspension (absorbance at 400 nm) (Fig. 9).

Control

The experiments of Referential Example were repeated using a diene-alpha-phosphatidyl choline represented by the following chemical formula, as disclosed in JP-A 6 067 489 the results were as shown in Fig. 9.

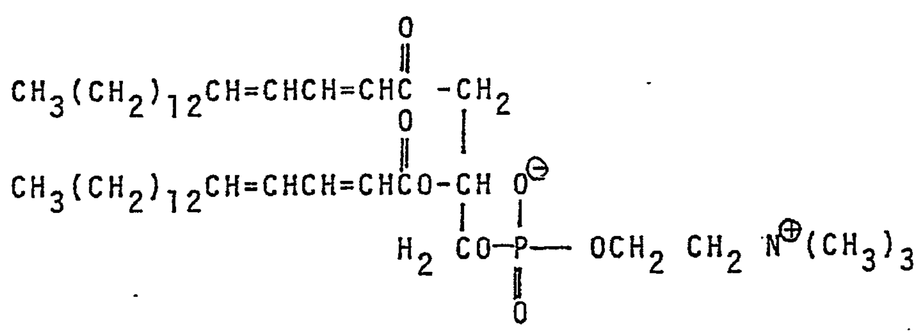

It is noted clearly from Fig. 9 that the decrease of the turbidity of the macromolecular liposome suspension or the destruction of the liposome due to the increase in the ethanol concentration was repressed in the polymeric beta-phosphatidyl choline as compared with that in the diene-alpha-phosphatidyl choline.

As described above, the present invention is directed to the polymerizable beta-glycerophospholipid represented by the general formula I and possessed of a symmetrical structure having conjugate triene groupe-containing acyl chains at the 1- and 3-positions of the glycerol skeleton. When this lipid is in the state forming a liposome, therefore, the polymerizable functional groups are not deviated. The monomer liposome formed from this lipid is easily polymerized. When the acyl chains possess polymerizable functional groups originating in eleostearic acid (conjugate trienes), they are easily polymerized by irradiation with the ultraviolet light. The macromolecular liposome resulting from the polymerization enjoys enhanced stability as compared with the liposome formed solely of natural phospholipid or the macromolecular liposome formed of the conventional asymmetrical polymerizable phosphatidyl choline. By depositing

medicinal substances, enzymes, hemoglobin, etc. on the macromolecular liposome formed from the polymerizable beta-glycerophospholipid of this invention, therefore, there can be obtained highly desirable medicines and drugs and artificial red blood cells.

The present invention is also directed to a method for the production of the polymerizable beta-glycerophospholipid represented by the general formula I, characterized by the steps of causing a 1,3-diglyceride represented by the general formula II to react with a 2-halo-2-oxo-1,3,2-dioxaphosphoran in the presence of a tertiary amine and subsequently causing the resultant reaction product to react with an amine or ammonia. The method of this invention, therefore, permits easy production of a polymerizable beta-glycerophospholipid I possessing a symmetrical structure like the aforementioned polymeric beta-phosphatidyl choline Ia. The 1,3-diglyceride to be used in the method for production according to the present invention can be obtained by the reaction of an unsaturated fatty acid anhydride with glycidol in the presence of a quaternary ammonium salt. The production of the polymerizable beta-phosphatidyl choline of the structural formula Ia from the eleostearic acid IIIa derived as the starting material from natural fatty acid is accomplished by preparing an eleostearic anhdyride VII by acid anhydridization using a haloformic ester, causing the acid anhydride to react with glycidol thereby forming 1,3-dieleostearoyl glyceride IIa, and causing the glyceride IIa to react with a 2-halo-2-oxo-1,3,2-dioxaphosphoran. Thus, the polymerizable functional groups of a naturally produced fatty acid can be utilized in their unaltered form. The method of this invention, therefore, has no need for the step of synthesizing a fatty acid possessing polymerizable functional groups by means of organic chemistry. Thus, it permits a generous reduction in the process and allows provision of polymerizable beta-glycerophospholipids enjoying outstanding properties mentioned above. Of course, the method for production according to this invention can be applied similarly effectively to the production using other fatty acids as the starting material. The present invention warrants efficient production of a polymerizable beta-glycerophospholipid possessing a symmetrical structure and giving rise to a liposome capable of efficient stable polymerization.

## Claims

1. A polymerizable beta-glycerophospholipid represented by the following formula I:

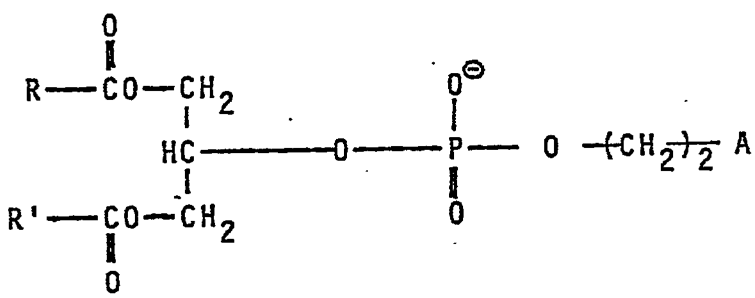

(I)

wherein R and R′ independently stand for an aliphatic hydrocarbon group possessing a conjugate triene as a polymerizable functional group and A stands for $N^{\oplus}R^1_3$ wherein $R^1$ stands for methyl group or ethyl group, or 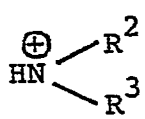 wherein $R^2$ and $R^3$ independently stand for hydrogen atom, methyl group, or ethyl group.

2. A polymerizable beta-glycerophospholipid according to Claim 1, wherein R and R′ independently stand for an aliphatic hydrocarbon group of 12 to 26 carbon atoms.

3. A polymerizable beta-glycerophospholipid according to Claim 1 or 2, wherein R and R′ each stand for $CH_3(CH_2)_3CH{=}CHCH{=}CHCH{=}CH(CH_2)_7$–.

4. A polymerizable beta-glycerophospholipid according to Claim 3, wherein A stands for $N^{\oplus}R^1_3$.

5. A polymerizable beta-glycerophospholipid according to Claim 4, wherein $R^1$ stands for a methyl group.

6. A method for the production of a polymerizable beta-glycerophospholipid represented by the following formula I:

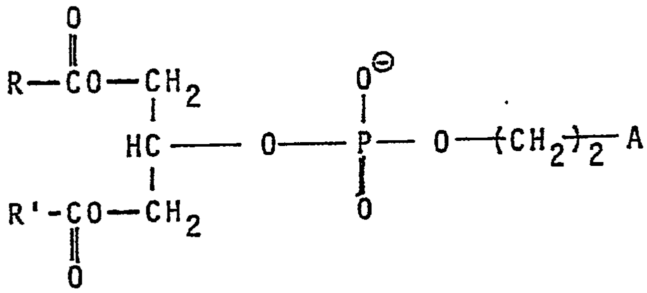 (I)

wherein R and R′ independently stand for an aliphatic hyddrocarbon group possessing a conjugate triene as a polymerizable functional group and A stands for $N^{\oplus}R^1_3$ wherein $R^1$ stands for methyl group or ethyl group, 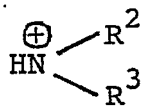 wherein $R^2$ and $R^3$ independently stand for hydrogen atom, methyl group, or ethyl group, characterized by the steps of causing a 1,3-diglyceride represented by the following general II:

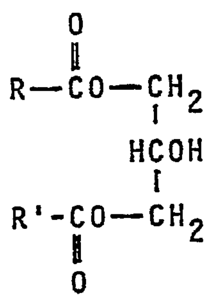 (II)

wherein R and R′ independently stand for an aliphatic hydrocarbon group possessing a conjugate triene as a polymerizable functional group, in an analogous manner to react with a 2-halo-2-oxo-1,3,2-dioxaphosphoran in the presence of a teritiary amine, and subsequently causing the resultant reaction product to react with an amine or ammonia.

7. A method according to Claim 6, wherein R and R′ independently stand for an aliphatic hydrocarbon group of 12 to 26 carbon atoms.

8. A method according to Claim 6 or 7, wherein said 1,3-diglyceride is represented by the following formula IIa:

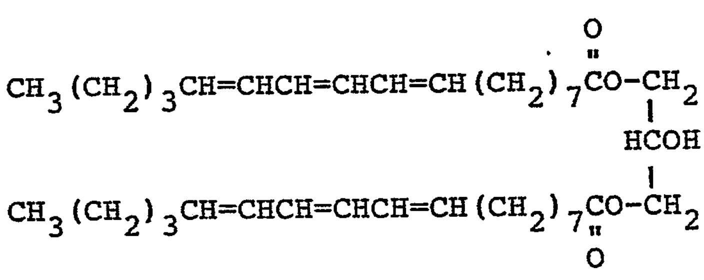 (IIa)

9. A method according to Claim 8, wherein said amine is trimethyl amine or triethyl amine.

10. A method according to Claim 8, wherein said amine is trimethyl amine.

11. A method according to Claim 7, wherein said 2-halo-2-oxo-1,3,2-dioxaphosphoran is 2-chloro-2-oxo-1,3,2-dioxaphosphoran.

12. A method according to Claim 7, wherein said 1,3-diglyceride represented by the general formula II is obtained by causing an unsaturated fatty acid anhydride to react with glycidol in the presence of a quaternary ammonium salt.

13. A method according to Claim 7, wherein said unsaturated fatty acid anhydride is eleostearic anhydride.

14. A method according to Claim 13, wherein said eleostearic anhydride is produced from the eleostearic acid contained in tung oil fatty acid by the use of a halo-formic acid ester.

**Patentansprüche**

1. Polymerisierbares β-Glycerophospholipid der folgenden Formel I:

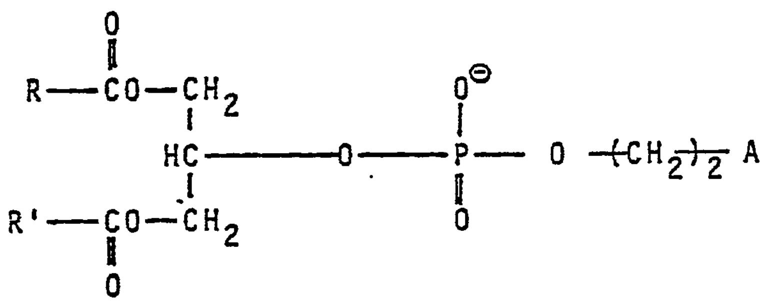

(I)

worin bedeuten:

R und R′ unabhängig voneinander eine aliphatische Kohlenwasserstoffgruppe mit einem konjugierten Trien als polymerisierbarer funktioneller Gruppe und A $N^{\oplus}R^1_3$ mit $R^1$ gleich einer Methyl- oder Ethylgruppe oder 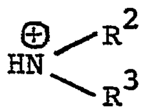 mit $R^2$ und $R^3$ unabhängig voneinander gleich einem Wasserstoffatom oder einer Methyl- oder Ethylgruppe.

2. Polymerisierbares β-Glycerophospholipid nach Anspruch 1, dadurch gekennzeichnet, daß R und R′ unabhängig voneinander für eine aliphatische Kohlenwasserstoffgruppe mit 12 bis 26 Kohlenstoffatomen stehen.

3. Polymerisierbares β-Glycerophospholipid nach Anspruch 1 oder 2, dadurch gekennzeichnet, daß R und R′ jeweils für $CH_3(CH_2)_3CH{=}CHCH{=}CHCH{=}CH(CH_2)_7-$ stehen.

4. Polymerisierbares β-Glycerophospholipid nach Anspruch 3, dadurch gekennzeichnet, daß A für $N^{\oplus}R^1_3$ steht.

5. Polymerisierbares β-Glycerophospholipid nach Anspruch 4, dadurch gekennzeichnet, daß $R^1$ für eine Methylgruppe steht.

6. Verfahren zur Herstellung eines polymerisierbaren β-Glycerophospholipids der folgenden Formel I:

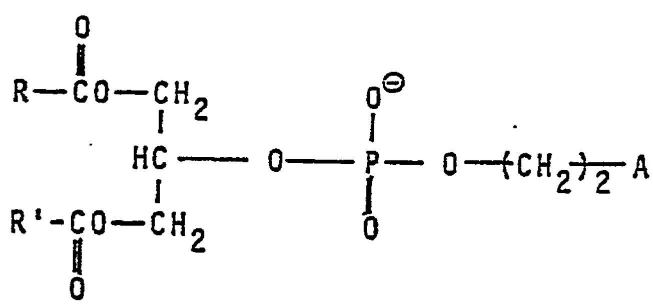

(I)

worin bedeuten:

R und R′ unabhängig voneinander eine aliphatische Kohlenwasserstoffgruppe mit einem konjugierten Trien als polymerisierbarer funktioneller Gruppe und A $N^{\oplus}R^1_3$ gleich einer Methyl- oder Ethylgruppe oder 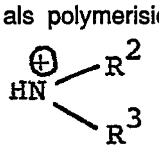 mit $R^2$ und $R^3$ unabhängig voneinander gleich einem Wasserstoffatom oder einer Methyl- oder Ethylgruppe, dadurch gekennzeichnet, daß man ein 1,3-Diglycerid der folgenden Formel II:

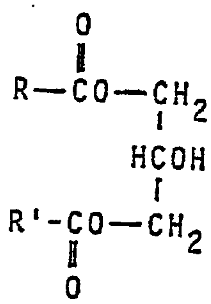

(II)

worin R und R′ unabhängig voneinander für eine aliphatische Kohlenwasserstoffgruppe mit einem konjugierten Trien als polymerisierbarer funktioneller Gruppe stehen, auf analoge Weise in Gegenwart eines tertiären Amins mit einem 2-Halogen-2-oxo-1,3,2-dioxaphosphoran und anschließend das gebildete Reaktionsprodukt mit einem Amin oder Ammoniak reagieren läßt.

7. Verfahren nach Anspruch 6, dadurch gekennzeichnet, daß R und R′ unabhängig voneinander für eine aliphatische Kohlenwasserstoffgruppe mit 12 bis 26 Kohlenstoffatomen stehen.

8. Verfahren nach Anspruch 6 oder 7, dadurch gekennzeichnet, daß das 1,3-Diglycerid durch folgende Formel IIa:

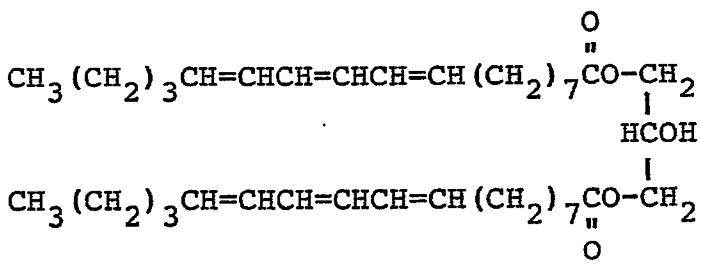

(IIa)

darstellbar ist.

9. Verfahren nach Anspruch 8, dadurch gekennzeichnet, daß das Amin aus Trimethylamin oder Triethlyamin besteht.

10. Verfahren nach Anspruch 8, dadurch gekennzeichnet, daß das Amin aus Trimethylamin besteht.

11. Verfahren nach Anspruch 7, dadurch gekennzeichnet, daß das 2-Halogen-2-oxo-1,3-2-dioxaphosphoran aus 2-Chlor-2-oxo-1,3,2-dioxaphosphoran besteht.

12. Verfahren nach Anspruch 7, dadurch gekennzeichnet, daß das 1,3-Diglycerid der allgemeinen Formel II durch Umsetzung eines ungesättigten Fettsäureanhydrids mit Glycid in Gegenwart eines quaternären Ammoniumsalzes erhalten wurde.

13. Verfahren nach Anspruch 7, dadurch gekennzeichnet, daß das ungesättigte Säureanhydrid aus Eleostearinsäureanhydrid besteht.

14. Verfahren nach Anspruch 13, dadurch gekennzeichnet, daß das Eleostearinsäureanhydrid aus der in Holzölfettsäure enthaltenen Eleostearinsäure mit Hilfe eines Halogenameisensäureesters erhalten wurde.

**Revendications**

1. Un bêta-glycérophospholipide polymérisable représenté par la formule I suivante:

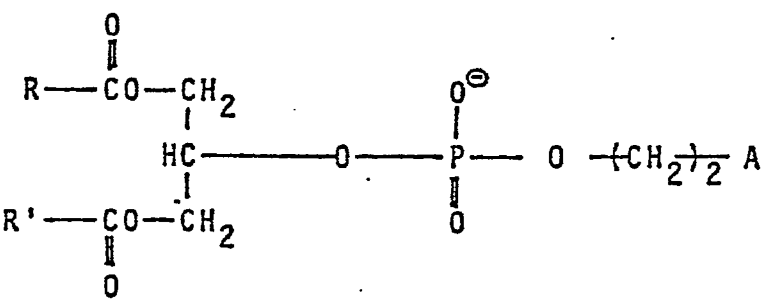

(I)

dans laquelle R et R′ représentent indépendamment un groupe hydrocarboné aliphatique possédant un

triène conjugué comme groupe fonctionnel polymérisable et A représente $N^{\oplus}R^1_3$ dans lequel $R^1$ représente un groupe méthyle ou éthyle ou 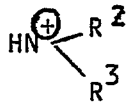 dans lequel $R^2$ et $R^3$ représentent indépendamment un atome d'hydrogène ou un groupe méthyle ou éthyle.

2. Un bêta-glycérophospholipide polymérisable selon la revendication 1, dans lequel R et R′ représentent indépendamment un groupe hydrocarboné aliphatique en $C_{12}$–$C_{16}$.

3. Un bêta-glycérophospholipide polymérisable selon la revendication 1 ou 2, dans lequel R et R′ représentent chacun $CH_3(CH_2)_3CH{=}CHCH{=}CHCH{=}CH(CH_2)_7$–.

4. Un bêta-glycérophospholipide polymérisable selon la revendication 3, dans lequel A représente $N^{\oplus}R^1_3$.

5. Un bêta-glycérophospholipide polymérisable selon la revendication 4, dans lequel $R^1$ représente un groupe méthyle.

6. Un procédé pour la fabrication d'un bêta-glycérophospholipide polymérisable représenté par la formule I suivante:

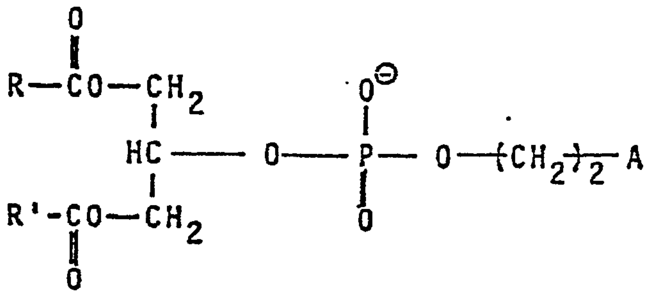

(I)

dans laquelle R et R′ représentent indépendamment un groupe hydrocarboné aliphatique possédant un triène conjugué comme groupe fonctionnel polymérisable et A représente $N^{\oplus}R^1_3$ dans lequel $R^1$ représente un groupe méthyle ou éthyle ou 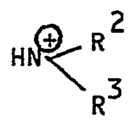 dans lequel $R^2$ et $R^3$ représentent indépendamment un atome d'hydrogène ou un groupe méthyle ou éthyle, caractérisé par les étapes de réaction d'un 1,3-diglycéride représenté par la formule générale II suivante:

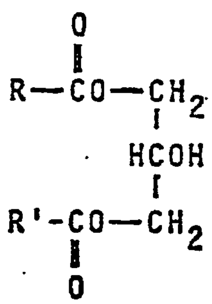

... (II)

dans laquelle R et R′ représentent indépendamment un groupe hydrocarboné aliphatique possédant un triène conjugué comme groupe fonctionnel polymérisable, de manière analogue avec un 2-halogéno-2-oxo-1,3,2-dioxaphosphorane en présence d'une amine tertiaire et ensuite de réaction du produit de réaction résultant avec une amine ou l'ammoniac.

7. Un procédé selon la revendication 6, dans lequel R et R′ représentent indépendamment un groupe hydrocarboné aliphatique en $C_{12}$–$C_{16}$.

8. Un procédé selon la revendication 6 ou 7, dans lequel ledit 1,3-diglycéride est représenté par la formule IIa suivante:

$$\begin{array}{ll} CH_3(CH_2)_3CH{=}CHCH{=}CHCH{=}CH(CH_2)_7\overset{O}{\overset{\|}{C}}O-CH2 & \\ \qquad\qquad\qquad\qquad\qquad\qquad\qquad\qquad\quad HCOH & (IIa) \\ CH_3(CH_2)_3CH{=}CHCH{=}CHCH{=}CH(CH_2)_7\underset{O}{\underset{\|}{C}}O-CH_2 & \end{array}$$

9. Un procédé selon la revendication 8, dans lequel ladite amine est la triméthylamine ou la triéthylamine.

10. Un procédé selon la revendication 8, dans lequel ladite amine est la triméthylamine.

11. Un procédé selon la revendication 7, dans lequel ledit 2-halogéno-2-oxo-1,3,2-dioxaphosphorane est le 2-chloro-2-oxo-1,3,2-dioxaphosphorane.

12. Un procédé selon la revendication 7, dans lequel ledit 1,3-diglycéride représenté par la formule générale II est obtenu en faisant réagir un anhydride d'acide gras insaturé avec le glycidol en présence d'un sel d'ammonium quaternaire.

13. Un procédé selon la revendication 7, dans lequel ledit anhydride d'acide gras insaturé est l'anhydride éléostéarique.

14. Un procédé selon la revendication 13, dans lequel ledit anhydride éléostéarique est produit à partir de l'acide éléostéarique contenu dans l'acide gras d'huile de tung en utilisant un ester halogénoformique.

FIG. 1

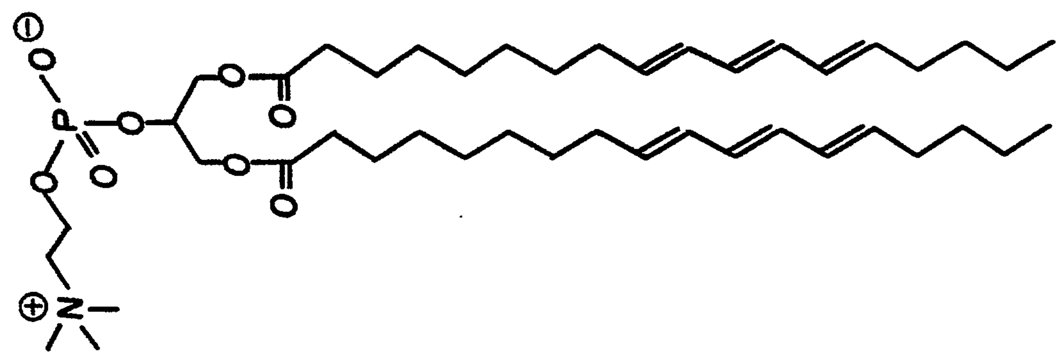

FIG. 2

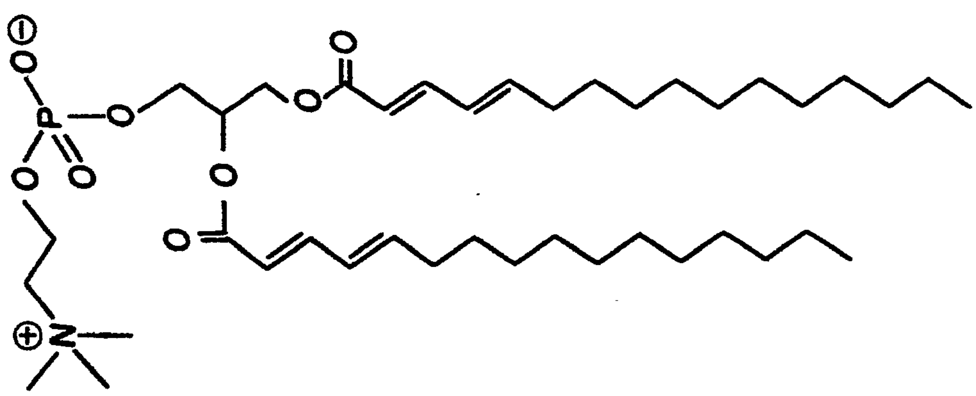

FIG. 3

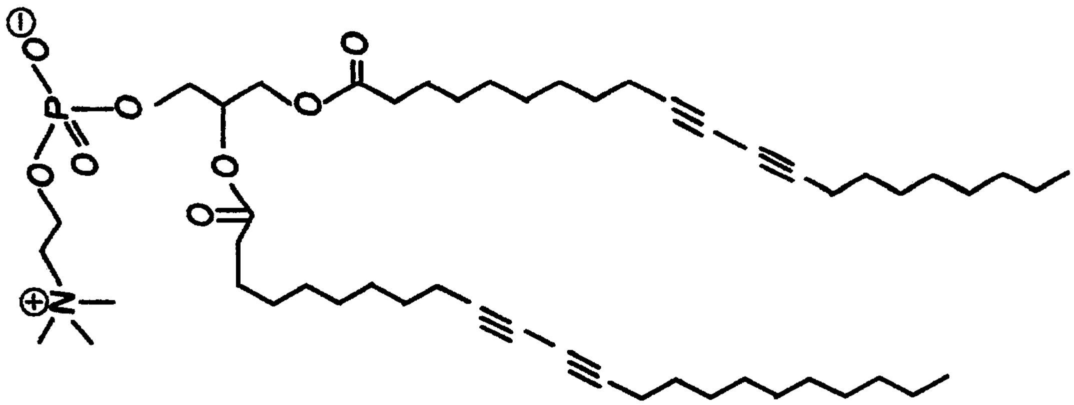

FIG. 4

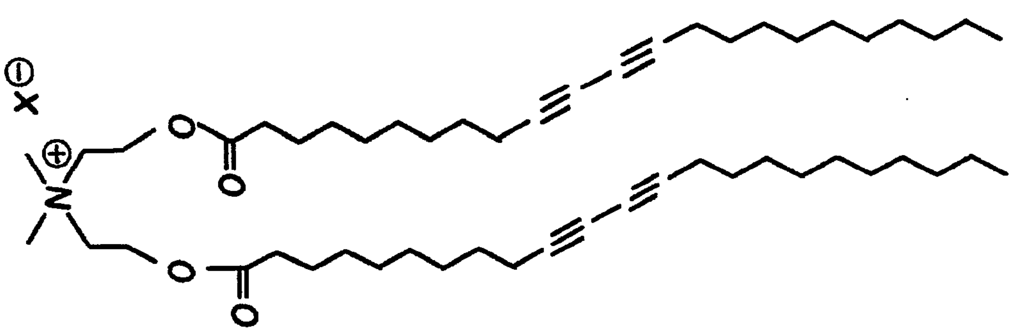

FIG. 5

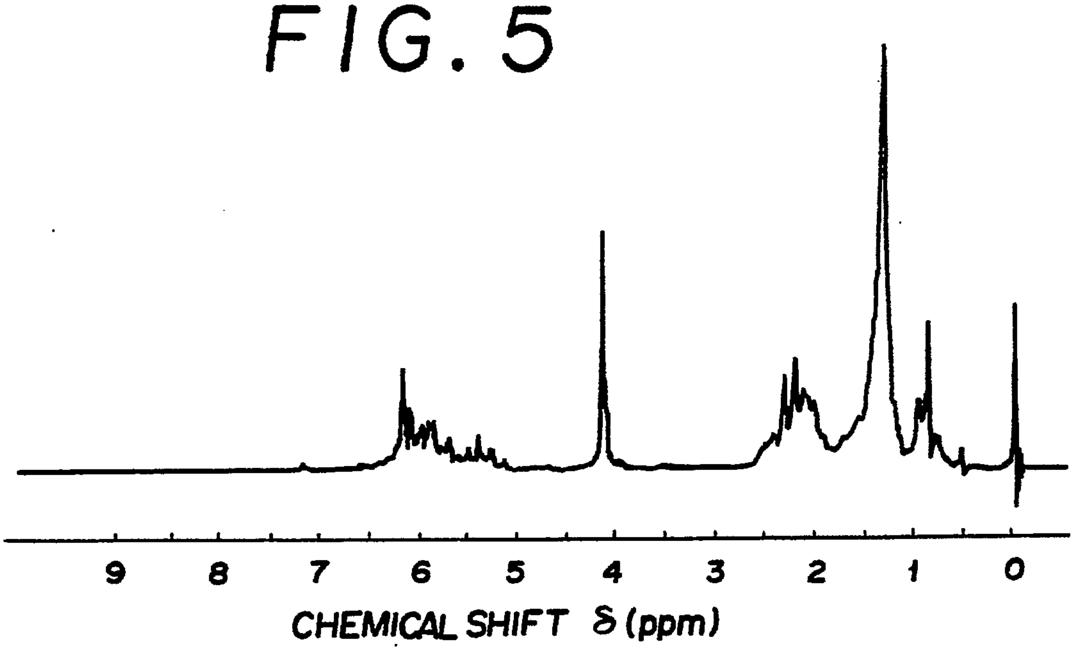
9
8
7
6
5
4
3
2
1
0
CHEMICAL SHIFT δ (ppm)

FIG. 6

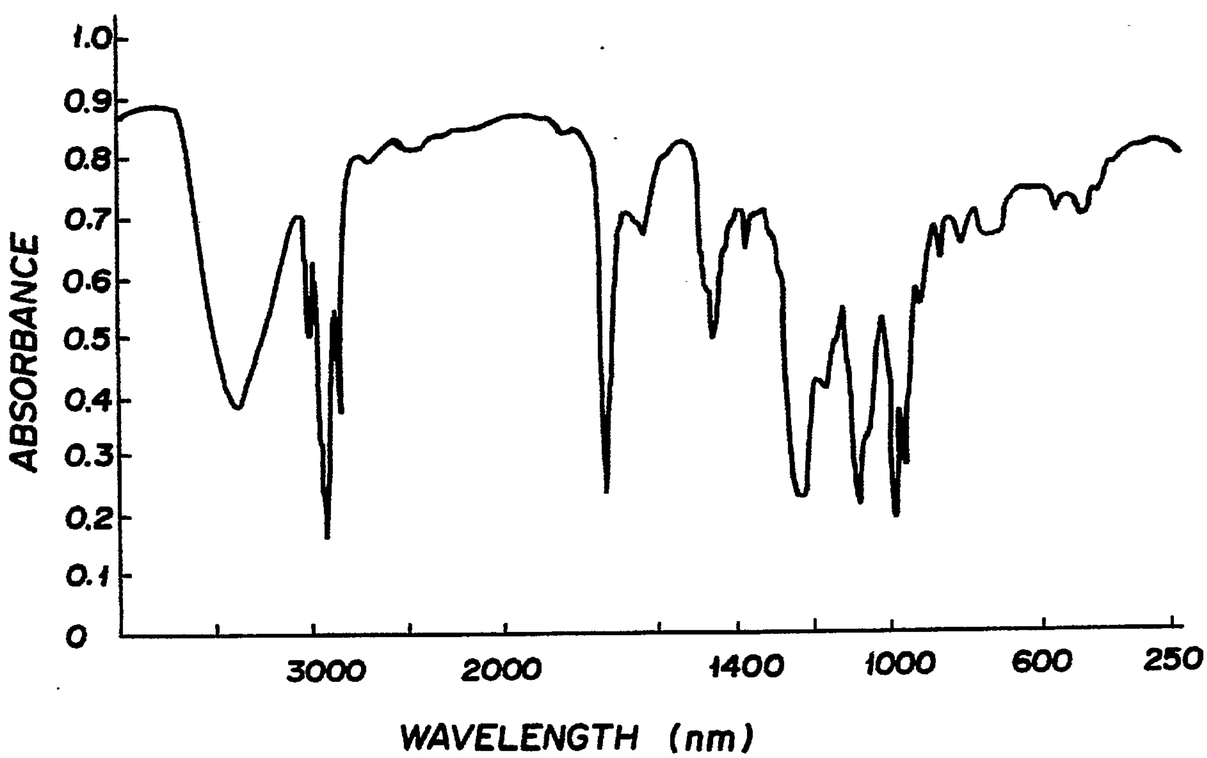
1.0
0.9
0.8
0.7
0.6
0.5
0.4
0.3
0.2
0.1
0
ABSORBANCE
3000
2000
1400
1000
600
250
WAVELENGTH (nm)

FIG. 7

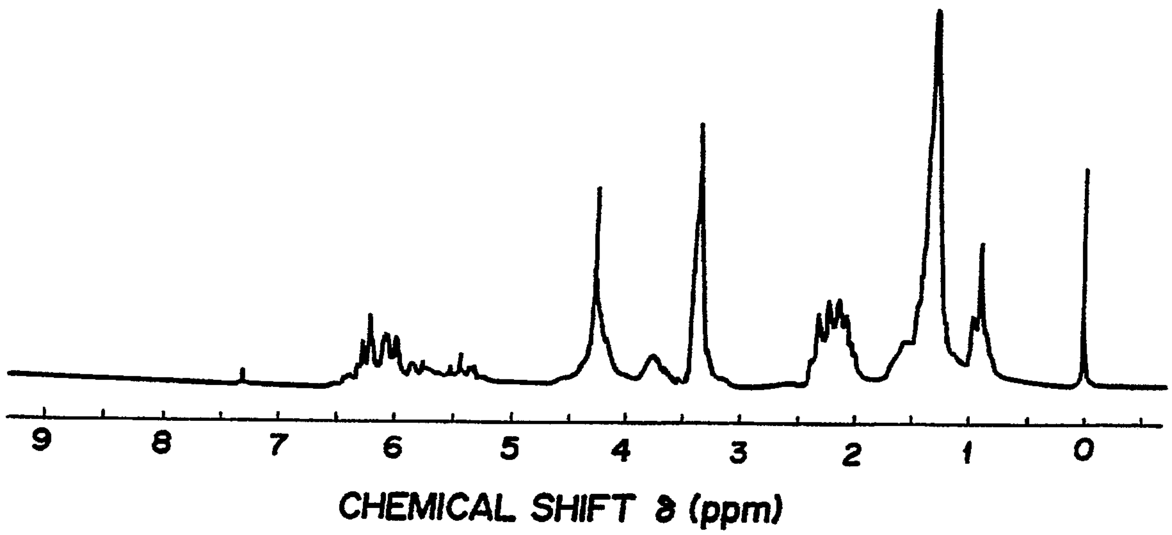
9
8
7
6
5
4
3
2
1
0
CHEMICAL SHIFT δ (ppm)

FIG. 9

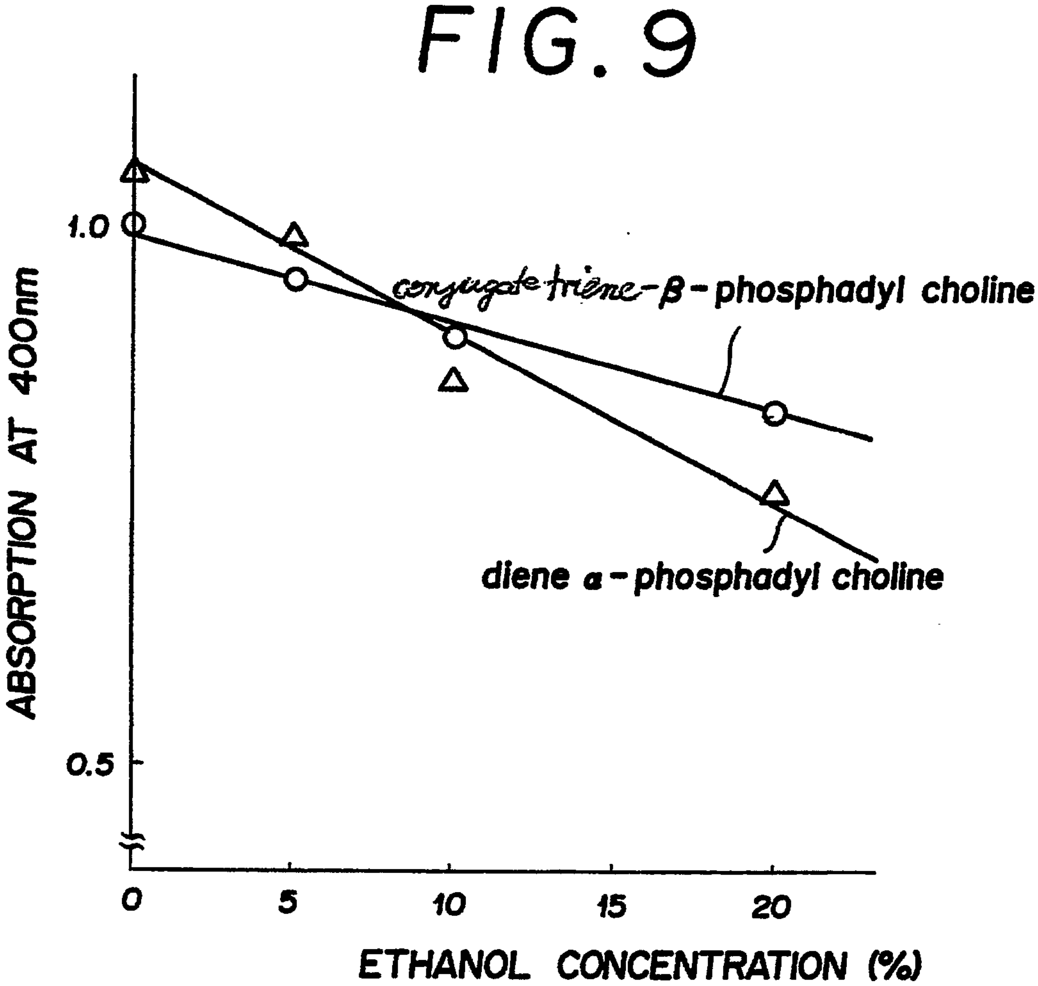
ABSORPTION AT 400nm
1.0
0.5
conjugate triene-β-phosphadyl choline
diene α-phosphadyl choline
0
5
10
15
20
ETHANOL CONCENTRATION (%)

FIG. 8

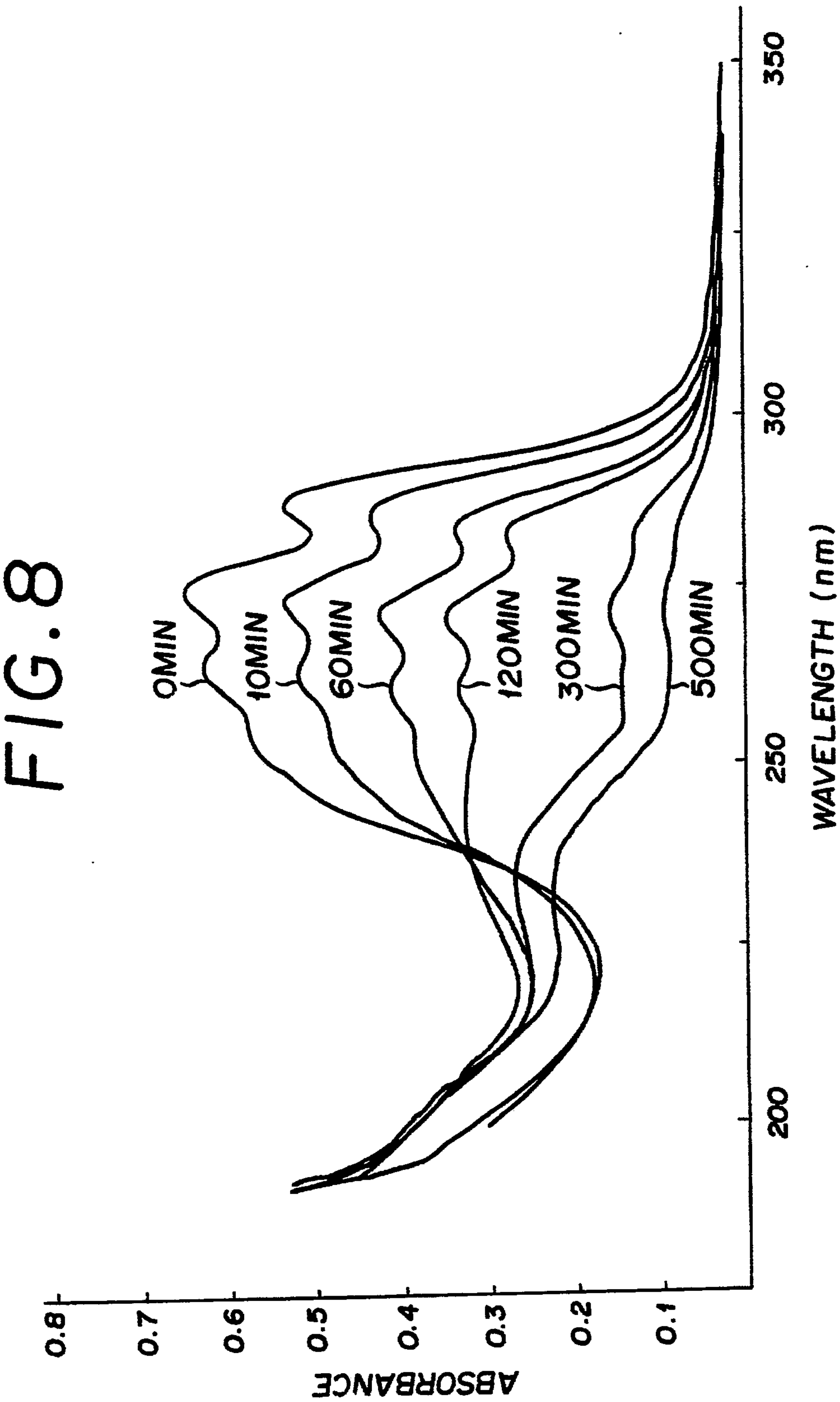
0MIN
10MIN
60MIN
120MIN
300MIN
500MIN
ABSORBANCE
0.8
0.7
0.6
0.5
0.4
0.3
0.2
0.1
WAVELENGTH (nm)
200
250
300
350